# EUROPEAN PATENT APPLICATION

(11) **EP 3 673 927 A1**
(43) Date of publication of application: **01.07.2020**
(21) Application number: 20150367.9
(22) Date of filing: 17.11.2016
(51) Int. Cl.: A61L 31/10

(54) **HYDROGEL COATED MESH**

(30) Priority: 18.11.2015 US 201562256853 P
(62) Divisional of application: 16805658.8
(71) Applicant: LifeCell Corporation, Madison, New Jersey 07940 (US)
(72) Inventor: Pomerleau, Ming F, Califon, NJ New Jersey 07830 (US); Owens, Rick T, Stewartsville, NJ New Jersey 08886 (US)
(74) Representative: Gunning, Jack Peter

(57) **Abstract**

The invention provides biocompatible mesh compositions and preparations thereof. Also featured are methods of treatment using the biocompatible mesh compositions.

## Description

### CROSS-REFERENCE TO RELATED APPLICATIONS

This application claims the benefit of U.S. Provisional Application No. 62/256,853, filed on November 18, 2015. The entire teachings of the aforementioned application are incorporated herein by reference.

### SUMMARY OF THE INVETION

Surgical meshes manufactured from synthetic materials are commonly used in hernia repair surgeries. Despite their widespread use, synthetic meshes are prone to numerous complications, which are in part due to the induction of a foreign body/inflammatory response.

There is a need to develop new meshes that can attenuate or minimize the foreign body responses or inflammatory reactions to implanted mesh materials.

The present invention is based on the discovery of a method of coating hyaluronic acids onto synthetic meshes. It is believed that a multi-layered molecular coating of hyaluronic acids (hydrogel) on the surface of synthetic meshes can significantly reduce or minimize the inflammatory reactions to implanted mesh materials. Based on these discoveries, a biocompatible mesh composition, and methods of making the same are disclosed herein.

One embodiment of the present invention is directed to a biocompatible mesh composition comprising: a mesh having a multi-layered molecular coating of hyaluronic acids, wherein the primary hydroxyl (-OH) groups of hyaluronic acids are cross-linked with the -OH containing groups on the mesh via a homobifunctional cross-linking agent, and the primary hydroxyl (-OH) groups of hyaluronic acids are also cross-linked to each other via the homobifunctional cross-linking agent.

Another embodiment of the present invention is directed to a process of making a biocompatible mesh composition, the method comprising:
i) treating a mesh with plasma to form a mesh with -OH containing groups on its surface;
ii) contacting the mesh with -OH containing groups with a solution containing hyaluronic acids and a homobifunctional cross-linking agent to form a biocompatible mesh composition in which the mesh has a multi-layered molecular coating of hyaluronic acids such that the primary hydroxyl (-OH) groups of hyaluronic acids are cross-linked with the - OH containing groups on the mesh via the homobifunctional cross-linking agent, and the primary hydroxyl (-OH) groups of hyaluronic acids are also cross-linked to each other via the homobifunctional cross-linking agent.

The present invention is further directed to a process of making a biocompatible mesh composition, the method comprising:
contacting a mesh with -OH containing groups on its surface with a solution containing hyaluronic acid and a homobifunctional cross-linking agent, to form a biocompatible mesh composition in which the mesh has a multi-layered molecular coating of hyaluronic acids such that the primary hydroxyl (-OH) groups of hyaluronic acids arc cross-linked with the -OH containing groups on the mesh via the homobifunctional cross-linking agent, and the primary hydroxyl (-OH) groups of hyaluronic acids are also cross-linked to each other via the homobifunctional cross-linking agent.

In one aspect, the process described herein comprises a further step of allowing the biocompatible mesh composition to dry.

In one aspect, in the process of the present invention, the plasma treatment is in the presence of allyl alcohol.

In one aspect, the homobifunctional cross-linking agent used in the present invention is butanediol diglycidyl ether (BDDE), 1, 2, 7, 8-diepoxyoctane (DEO), glycerol diglycidyl ether, or divinyl sulfone (DVS). In one embodiment, the homobifunctional cross-linking agent used in the present invention is butanediol diglycidyl ether (BDDE).

In one aspect, the mesh is polystyrene, polyethylene, polypropylene, polyethylene terephthalate, polytefrafluoroethylene, polylactide, cellulose or silk. In one embodiment, the mesh is polypropylene. In one embodiment, the mesh is cellulose. In one embodiment, the mesh is silk.

In one aspect, the -OH containing groups on the mesh are represented by -RCH₂OH, wherein R is C₁-C₆ alkylene or R is absent.

In one aspect, the hyaluronic acid used in the present invention has a molecular weight in a range of from about 350,000 daltons to about 2,000,000 daltons.

In the foregoing biocompatible mesh composition embodiments, the mesh is silk and the homobifunctional cross-linking agent is butanediol diglycidyl ether (BDDE). Alternatively, the mesh is cellulose and the homobifunctional cross-linking agent is butanediol diglycidyl ether (BDDE). Still alternatively, the mesh is polypropylene and the homobifunctional cross-linking agent is butanediol diglycidyl ether (BDDE).

In the foregoing biocompatible mesh composition embodiments, the molar ratio between the hyaluronic acid and the homobifunctional cross-linking agent is 20:1 to 1:1 (for example, 15:1 to 1:1).

In one aspect, the mesh used in the biocompatible mesh composition of the present invention is in the form of a flexible sheet.

The biocompatible mesh compositions of the present invention include any compositions formed by the methods as described above. In the above described compositions and methods, the recited embodiments can be combined in any combination desired.

Unless otherwise defined, all technical and scientific terms used herein have the same meaning as commonly understood by one of ordinary skill in the art to which this invention pertains. Although methods and materials similar or equivalent to those described herein can be used in the practice or testing of the present invention, suitable methods and materials arc described below. All publications, patent applications, patents, and other references mentioned herein are incorporated by reference in their entirety. In case of conflict, the present specification, including definitions, will control. In addition, the materials, methods, and examples are illustrative only and not intended to be limiting.

The details of one or more embodiments of the invention are set forth in the accompanying drawings and the description below. Other features, objects, and advantages of the invention will be apparent from the description and drawings, and from the claims.

### BRIEF DESCRIPTION OF THE DRAWINGS

Figure 1 is a flow chart showing a process of making a biocompatible mesh composition, according to certain embodiments.
Figure 2A is a side view of a mesh composition, according to certain embodiments.
Figure 2B is a side view of another mesh composition, according to certain embodiments.

### DETAILED DESCRIPTION OF EXEMPLARY EMBODIMENTS

The materials and methods provided herein can be used to make a biocompatible mesh composition that can be implanted into a damaged or defective organ or tissue to facilitate the repair of the damaged or defective organ or tissue. As used herein, a "biocompatible" composition is one that has the ability to support cellular in-growth and activity necessary for complete or partial tissue regeneration, but does not stimulate a significant local or systemic inflammatory or immunological response in the host. As used herein, a "significant local or systemic inflammatory or immunological response in the host" is a local or systemic inflammatory or immunological response that partially or completely prevents tissue regeneration by a composition of the invention.

The term "about" when used before a numerical designation, e.g., temperature, time, amount, and concentration, including range, indicates approximations which may vary by ± 10%, 5% or 1%.

One embodiment of the present invention is directed to a biocompatible mesh composition comprising: a mesh having a multi-layered molecular coating of hyaluronic acids, wherein the primary hydroxyl (-OH) groups of hyaluronic acids arc cross-linked with the -OH containing groups on the mesh via a homobifunctional cross-linking agent, and the primary hydroxyl (-OH) groups of hyaluronic acids are also cross-linked to each other via the homobifunctional cross-linking agent.

Another embodiment of the present invention is directed to a process of making a biocompatible mesh composition, the method comprising:
i) treating a mesh with plasma to form a mesh with -OH containing groups on its surface;
ii) contacting the mesh with -OH containing groups with a solution containing hyaluronic acids and a homobifunctional cross-linking agent to form a biocompatible mesh composition in which the mesh has a multi-layered molecular coating of hyaluronic acids such that the primary hydroxyl (-OH) groups of hyaluronic acids are cross-linked with the - OH containing groups on the mesh via the homobifunctional cross-linking agent, and the primary hydroxyl (-OH) groups of hyaluronic acids are also cross-linked to each other via the homobifunctional cross-linking agent.

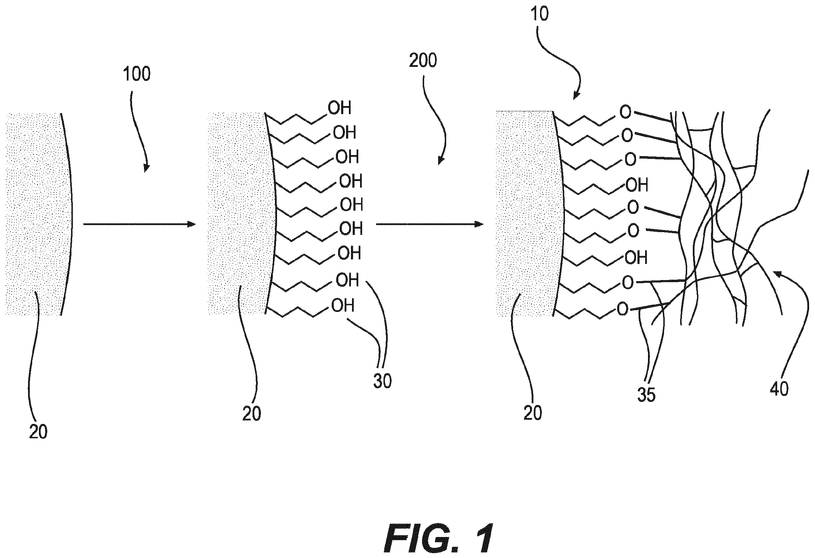

Figure 1 is a flow chart showing a process of making a biocompatible mesh composition, according to certain embodiments. As shown in Figure 1, a mesh 20 can be treated with plasma (Step 100) to form a mesh with -OH containing groups 30 on its surface. Next, the mesh with -OH containing groups can be applied to a solution containing hyaluronic acids 40 and a homobifunctional cross-linking agent (Step 200) to form a biocompatible mesh composition 10.

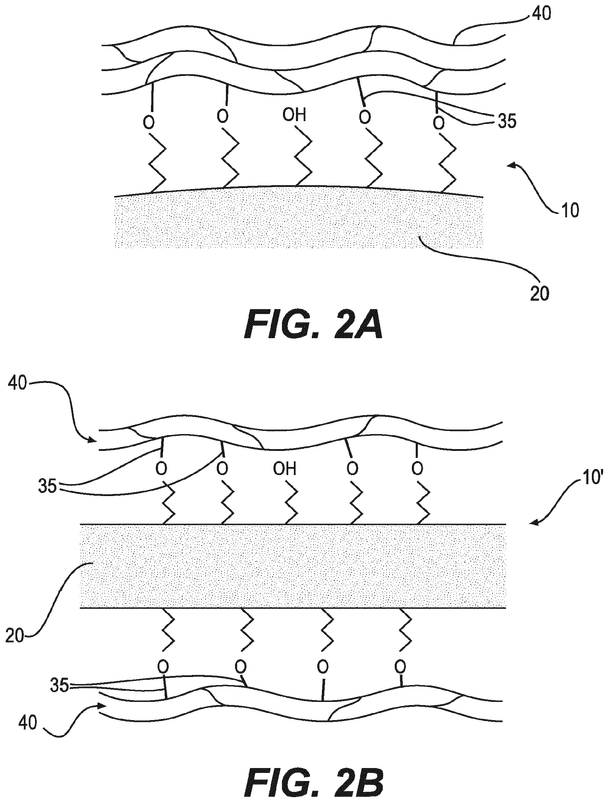

It will be appreciated that the coating of hyaluronic acid can be applied to part or all of the mesh 20. For example, Figure 2A is a side view of a mesh composition, according to certain embodiments, wherein the coating 40 is applied to one side of the mesh 10. And Figure 2B is a side view of another mesh composition 10', according to certain embodiments, wherein the coating 40 is applied to both sides or the entire surface of the mesh 10'.

### I. Composition Components

### Hyaluronic Acids

The composition of the invention is made by coating multi-layered hyaluronic acids (HA) on the surface of a mesh substrate. The coating of implantable medical devices with the compositions provided herein in order to attenuate a foreign body response is also contemplated. Examples of suitable devices include, without limitation, artificial joints, vascular grafts, artificial valves, cardiac pacemakers, cardiac defibrillators, muscle stimulators, neurological stimulators, cochlear implants, monitoring devices, drug pumps and left ventricular assist devices.

Hyaluronic acid is a naturally occurring polymer found in the extracellular matrix of tissue, vitreous humor, and cartilage. The total quantity of HA found in a 70-kg person is approximately 15 g, and its average turnover rate is 5 g/d. Approximately 50% of the total quantity of HA in the human body is concentrated in the skin, and it has a half-life of 24-48 hours. Hyaluronic acid is a polysaccharide that consists of repeating monomers (glucuronic acid and N-acetylglucosamine disaccharide units) linked together in a linear fashion through β-1,4 glycosidic bonds. The formula of HA is shown as below: 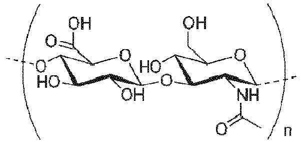 wherein n is the number of repeating units. Generally, hyaluronic acids used herein have a molecular weight of from about 350,000 Daltons to about 3.0 MDa (mega Dalton). In some embodiments, the molecular weight is from about 350,000 Daltons to about 2,000,000 daltons. In some embodiments, the molecular weight is from about 0.6 MDa to about 2.6 MDa, and in yet another embodiment, the molecular weight is from about 1.4 MDa to about 1.6 MDa. In some embodiments, the molecular weight is about 0.7 MDa and in yet another embodiment, the molecular weight is about 1.6 MDa. In some embodiments, the molecular weight is about 2.6 MDa.

Suitable derivatives of HA that may be used in the invention will be known to the skilled artisan, and are described, for example, in U.S. Pat. No. 4,851,521. These include partial esters of hyaluronic acid with alcohols of the aliphatic, araliphatic, cycloaliphatic and heterocyclic series and salts of such partial esters with inorganic or organic bases. The derivatives of HA also include deacylated HA which have free amino (-NH₂) groups.

### Mesh

Any biocompatible mesh, *e.g.,* a surgical mesh, can be used in the biocompatible compositions of the present invention.

Surgical meshes are materials that are available in many forms (*e.g.,* a flexible sheet) and have been produced from a variety of synthetic and natural materials. Meshes can be broadly classified according to filament structure, pore size and weight. Filament structure can be monofilament, multifilament or multifilament fibers formed from monofilament materials. Mesh pore sizes can range from between about 200µ to about 5000µ. Small pore sizes, *e.g.,* 1000µ or less, are typical of heavyweight meshes, while larger pore sizes, *e.g.,* greater than 1000µ are characteristic of lightweight meshes. Mesh weight is expressed as g/m², with heavyweight meshes having densities of about 80-100 g/m² and lightweight meshes having densities in the range of 25-45 g/m². Suitable surgical meshes can include woven, knitted, molded, unitary, or multi-component materials, as well as meshes formed using other processes.

The mesh can be made of a non-absorbable material, an absorbable material or a material that is a combination of both non-absorbable and absorbable materials. "Absorbable material" is defined herein as any material that can be degraded in the body of a mammalian recipient by endogenous hydrolytic, enzymatic or cellular processes. Depending upon the particular composition of the material, the degradation products can be recycled via normal metabolic pathways or excreted through one or more organ systems. A "non-absorbable material" is one that cannot be degraded in the body of a mammalian recipient by endogenous hydrolytic, enzymatic or cellular processes.

Polymers used to make non-absorbable meshes include polypropylene, polyester, *i.e.,* polyethylene terephthalate, or polytetrafluoroethylene (PTFE). Examples of commercially available polypropylene meshes include: Marlex™ (CR Bard, Inc., Cranston R.I.), Visilex® (CR Bard, Inc., Cranston R.I.), PerFix® Plug (CR Bard, Inc., Cranston R.I.), Kugel™ Hernia Patch (CR Bard, Inc., Cranston R.I.), 3DMax (CR Bard, Inc., Cranston R.I.), Prolene™ (Ethicon, Inc., Somerville, N.J.), Surgipro™ (Autosuture, U.S. Surgical, Norwalk, Conn.), Prolite™ (Atrium Medical Co., Hudson, N.H.), Prolite Ultra™ (Atrium Medical Co., Hudson, N.H.), Trelex™ (Meadox Medical, Oakland, N.J.), and Parietene® (Sofradim, Trévoux, France). Examples of commercially available polyester meshes include Mersilene™ (Ethicon, Inc., Somerville, N.J.) and Parietex® (Sofradim, Trevoux, France). Examples of commercially available PTFE meshes include Goretex® (W. L. Gore & Associates, Newark, Del.), Dualmesh® (W. L. Gore & Associates, Newark, Del.), Dualmesh® Plus (W. L. Gore & Associates, Newark, Del.), Dulex® (CR Bard, Inc., Cranston R.I.), and Reconix® (CR Bard, Inc., Cranston R.I.).

Absorbable meshes are also available from commercial sources. Polymers used to make absorbable meshes can include polyglycolic acid (Dexon™, Syneture™, U.S. Surgical, Norwalk, Conn.), poly-1-lactic acid, polyglactin 910 (Vicryl™, Ethicon, Somerville, N.J.), or polyhydroxylalkaoate derivatives such as poly-4-hydroxybutyrate (Tepha, Cambridge, Mass.).

Composite meshes, *i.e.,* meshes that include both absorbable and non-absorbable materials can be made either from combinations of the materials described above or from additional materials. Examples of commercially available composite meshes include polypropylene/PTFE: Composix® (CR Bard, Inc., Cranston R.I.), Composix® E/X (CR Bard, Inc., Cranston R.I.), and Ventralex® (CR Bard, Inc., Cranston R.I.); polypropylene/cellulose: Proceed™ (Ethicon, Inc., Somerville, N.J.); polypropylene/Seprafilm®: Sepramesh® (Genzyme, Cambridge, Mass.), Sepramesh® TP (Genzyme, Cambridge, Mass.); polypropylene/Vicryl: Vypro™ (Ethicon, Somerville, N.J.), Vypro™ II (Ethicon, Somerville, N.J.); polypropylene/Monocryl(poliglecaprone): Ultrapro® (Ethicon, Somerville, N.J.); and polyester/collagen: Parietex® Composite (Sofradim, Trevoux, France).

Examples of meshes used in the present invention include, but are not limited to, polystyrene, polyethylene, polypropylene, polyethylene terephthalate, polytefrafluoroethylene, or polylactide. Meshes containing -OH groups on their surfaces (such as cellulose or silk) can also be used in the present invention. Another example is synthetic co-polymers in which one of the monomers contains -OH in the side chain.

### II. Biocompatible Mesh Composition Preparation

The biocompatible mesh compositions described herein can be made by cross-linking HA with -OH containing groups on the surface of the mesh, as well as crossing-linking HA themselves to form a hydrogel. If the mesh used in the present invention does not contain - OH groups on its surface, it can be subjected to plasma-treatment in the presence of an alcohol resulting in surface functionalization wih hydroxyl (-OH) groups.

### Plasma Treatment

The use of plasma techniques is familiar to those of skill in the art (see, for example, Garbassi F. et al, "Polymer Surfaces, from Physics to Technology", Wiley, Chichester, 6, 1994, and N. Inagaki "Plasma Surface Modification and Plasma Polymerization, Technomic Publishing Company, Lancaster, 1996). In the present invention, the plasma treatment process may be any process that is capable of causing hydroxyl to be incorporated onto the surface of the mesh resulting in reactive -OH-containing groups, including direct as well as remote plasma treatment methods.

The plasma treatment can be performed at various conditions. Generally, plasma is generated by a plasma frequency of from about 1 kHz to about 2,500 MHz. In various embodiments, the plasma is generated by a plasma frequency of from about 10 kHz to about 14 MHz, or more particularly, from about 40 kHz to about 14 MHz. In one embodiment, the plasma treatment is performed at a power of from about 62 watts to about 700 watts, such as about 380 watts. In one embodiment, the gas flow rate for the plasma treatment is from about 0.9 standard liters per minute to about 1.2 standard liters per minute, such as about 1.08 standard liters per minute. The plasma is generated at a pressure of from about 1 mTorr to about 2,000 mTorr. In one embodiment, the plasma is generated at a pressure of from about 50 mTorr to about 500 mTorr with about 250 mTorr as nominal pressure. Alternatively or additionally, the plasma is generated at an atmospheric pressure. In various embodiments, the plasma is generated at a pressure of from about 680 Torr to about 1,520 Torr, from about 720 Torr to about 800 Torr, or about 760 Torr. The plasma treatment is performed at a temperature of from about 16 degrees Celsius (° C.) to about 100° C. In one embodiment, the plasma treatment is performed at a temperature of from about 25° C. to about 45° C. The expandable member is exposed to the plasma treatment for from about 10 seconds to about 1,000 seconds. In one embodiment, the expandable member is exposed to the plasma treatment for from about 40 seconds to about 420 seconds, such as at least about 75 seconds. In one embodiment, the expandable member is exposed to the supplied gas for about 15 minutes or less after the plasma treatment is complete.

In accordance with the disclosed subject matter, the plasma treatment is performed by supplying a plasma treatment gas to the processing chamber. Many gases or mixture of gases can be used in the invention. For example, the plasma treatment gas includes, but is not limited to, an inert gas, a nitrating gas, or combinations thereof. Suitable inert gases include noble gases, such as argon, neon, xenon, helium, radon, and combinations thereof. A nitrating gas can include nitrogen containing compounds include, but are not limited to, nitrogen, nitrogen oxides, activated-dinitrogen, ammonia, hydrazine, methylhydrazine, dimethylhydrazine, t-butylhydrazine, phenylhydrazine, azoisobutane, ethylazide, tert-butylamine, allylamine, derivatives thereof, and combinations thereof. In addition, the plasma treatment gas can include oxygen, ozone, hydrogen peroxide, carbon dioxide, carbon monoxide, carbon tetrafluoride, water vapor, allyl alcohol, methane, and a combination thereof. The use of these gases and a combination thereof can facilitate plasma formation form a plasma with high density and high uniformity. In one embodiment, the plasma treatment gas is argon. Alternatively, or additionally, the plasma treatment gas is a mixture of argon and oxygen. Oxygen has higher ionization energy than argon, and use of oxygen in addition to argon can result in more uniform plasma than use of argon by itself. The ratio of argon:oxygen supplied at the discharge space is ranged from about 10:90 to about 90:10 by volume. In one embodiment, the ratio of argon:oxygen is about 50:50 by volume. The plasma treatment gas can be supplied at a gas flow rate of from about 0.9 to about 1.2 standard liters per minute (SLPM), such as about 1.08 SLPM.

The plasma may be sustained over the full treatment time or may be administered in pulses. Plasma treatment and plasma polymerization are the two main routes available for producing -OH groups on a mesh substrate. In the case of plasma treatment, a monomer gas, most commonly allyl alcohol, is introduced together with an energized reactive gas onto the mesh substrate, resulting in the chemical insersion of -OH functional groups on the substrate. In the case of plasma polymerization, monomers used are methanol, ethanol, isopropyl alcohol, allyl alcohol, methylbutylnol, propan-1-ol, propargyl alcohol, furfuryl alcohol and isobutyl alcohol. More detailed information regarding plasma treatment and plasma polymerization can be found in Siow et al., Plasma Process. Polym. 2006, 3, 392-418.

### Cross-linking

The purpose of the plasma treatment is to create a high surface concentration of covalently attached hydroxyl groups. The hydroxyl groups can then be chemically cross-linked (e.g., covalently linked) to hyaluronic acid or a derivative thereof, in the presence of a cross-linking agent.

"Cross-linking agents" used herein contain at least two reactive functional groups that create covalent bonds between two or more molecules. The cross-linking agents can be homo-bifunctional (i.e. have two reactive ends that are identical) or hetero-bifunctional (i.e. have two different reactive ends). The chemistries available for such linking reactions include, but are not limited to, reactivity with sulfhydryl, amino, carboxyl, diol, aldehyde, ketone, or other reactive groups using electrophilic or nucleophilic chemistries, as well as photochemical cross-linkers using alkyl or aromatic azido or carbonyl radicals. Examples of cross-linking agents include, without limitation, glutaraldehyde, carbodiimides, bisdiazobenzidine, and N-maleimidobenzoyl-N-hydroxysuccinimide ester. Cross-linking agents are widely available from commercial sources (e.g., Pierce Biotechnology (Rockford, Ill.); Invitrogen (Carlsbad, Calif.); Sigma-Aldrich (St. Louis, Mo.); and US Biological (Swampscott, Mass.)).

In the present invention, the hydroxyl groups of the mesh are cross-linked with the active primary hydroxyl groups of hyaluronic acid via a chemical cross-linking agent. Furthermore, as illustrated in Figure 1, two or more polymer chains of hyaluronic acid are also cross-linked to each other to form a multi-layered molecular HA coating (*i.e.,* hydrogel) via a cross-linking agent. Additionally, intramolecular cross-linking of HA may also occur within individual HA polymer chains during this process. Intramolecular cross-linking of HA however is not envisioned to contribute to the multi-layering of HA or coupling to the mesh. Such cross-linking is differentiated from intramolecular or intermolecular dehydration, which results in lactone, anhydride, or ester formation within a single polymer chain or between two or more chains. The term "cross-linked" is also intended to refer to hyaluronic acid covalently linked to a cross-linking agent. In some embodiments, the term "cross-linked" also refers to covalently modified hyaluronic acid.

In one embodiment, a homo-bifunctional cross-linking agent is used, such as butanediol diglycidyl ether (BDDE), 1, 2, 7, 8-diepoxyoctane (DEO), glycerol diglycidyl ether, or divinyl sulfone (DVS).

It is noted that a hetero-bifunctional cross-linking agent (e.g., 1-Ethyl-3-(3-dimethylaminopropyl)carbodiimide (EDC) or N,N'-dicyclohexylcarbodiimide (DCC)) can also be used in the present invention. Yet, in order to form a multi-layered molecular HA coating, hyaluronic acid needs to be chemically modified to create reactive sites for cross-linking. For example, HA can be partially deacetylated to creat active amnio (-NH₂) groups. As such, the active carboxyl (-COOH) groups of HA are able to cross-link with the amino groups of HA in order to form a multi-layered coating. The modified HA can then be crosslinked to a mesh that contains either -COOH or -NH₂ functionality.

In one aspect, hyaluronic acid is prepared as aqueous solution and added to the -OH functionalized mesh together with a cross-linking agent. In certain embodiments, the cross-linked HA form gels. In certain embodiments, the hyaluronic acid is hydrated for between about one minute and about 60 minutes prior to cross-linking. In other embodiments, the hyaluronic acid is hydrated for between about one hour and about 12 hours prior to cross-linking. In certain embodiments, the hyaluronic acid is hydrated for about one hour and in yet another embodiment the hyaluronic acid is allowed to hydrate for about two hours prior to cross-linking. In certain embodiments, the hyaluronic acid is hydrated for about three hours and in yet another embodiment the hyaluronic acid is allowed to hydrate for four hours prior to cross-linking.

The mixed solution is allowed to sit for a certain period time before a desired biocompatible mesh compostion is formed. The period time is 10 minutes, 20 minutes, 30 minutes, 40 minutes, 50 minutes, 1 hour, 2 hours, 3 hours, 4 hours, or 5 hours. During the sitting, the mixed solution can be maintained at 20 °C, or heated at at a tempearature of about 25 °C, 30 °C, 35 °C, 40 °C, 45 °C, or 50 °C.

Prior to addition of the HA, the aqueous solution is adjusted to the desired pH. In one embodiment, the aqueous solution has a pH > 7. In certain embodiments, the solution has a pH of about 9, or about 10, or about 11, or about 12 or about 13, or greater than 13. Typically, the solution comprises water and can optionally comprise phosphate buffered saline (PBS) or tris(hydroxymethyl)aminomethane (Tris) buffer. The buffer can be selected based on the desired pH of the composition. For example, PBS can be used for compositions at a pH of about 7, whereas Tris can be used for compositions having a higher pH of about 9 or about 10. In some embodiments, the pH is from between about 9 and about 13. In some embodiments, the pH is at least about 13. In some embodiments, the pH is adjusted with the appropriate amount of a suitable base, such as Na₂CO₃ or NaOH to reach the desired pH. In some embodiments, the concentration of base is from about 0.00001 M to about 0.5 M. In some embodiments, the concentration of base is from about 0.1 M to about 0.25 M. In some embodiments, the concentration of base is about 0.25 M.

In one embodiment, the composition during the cross-linking comprises from about 5 mg/mL to about 50 mg/mL hyaluronic acid, before cross-linking. In another embodiment, the composition during the cross-linking comprises about 25 mg/mL to about 50 mg/mL hyaluronic acid, before cross-linking.

It has also been discovered that the concentration of cross-linking agent, e.g., BDDE, used during cross-linking contributes to the quality of the compositions comprising cross-linked hyaluronic acid and, ultimately, to improve certain properties of the biocompatible mesh compositions.

In order to yield a desired biocompatible mesh compostion, the molar ratio between hyaluronic acid and a cross-linking agent (e.g., BDDE) used is between 1:1 and 20:1. In one embodiment, the molar ratio between hyaluronic acid and a cross-linking agent (e.g., BDDE) used is 1:1. In another embodiment, the molar ratio between hyaluronic acid and a cross-linking agent (e.g., BDDE) used is 5:1. In another embodiment, the molar ratio between hyaluronic acid and a cross-linking agent (e.g., BDDE) used is 8:1. In another embodiment, the molar ratio between hyaluronic acid and a cross-linking agent (e.g., BDDE) used is 10:1. In another embodiment, the molar ratio between hyaluronic acid and a cross-linking agent (e.g., BDDE) used is 12:1. In another embodiment, the molar ratio between hyaluronic acid and a cross-linking agent (e.g., BDDE) used is 15:1. In another embodiment, the molar ratio between hyaluronic acid and a cross-linking agent (e.g., BDDE) used is 17:1. In another embodiment, the molar ratio between hyaluronic acid and a cross-linking agent (e.g., BDDE) used is 20:1.

In certain aspects, hyaluronic acid is cross-linked or covalently modified to form compositions comprising substantially cross-linked hyaluronic acid. In certain embodiments, the amount of cross-linking agent incorporated therein, or cross-link density, should be sufficiently high such that the composition formed thereby has a prolonged degradation profile. However , it should not be so high that the resulting composition loses its biocompatility and other biological benefits.

### Washing and Drying

After the cross-linking process is completed, the obtained biocompatible mesh composition can be subject to a further step of drying. The drying can be accomplished by air drying or vacuum drying, at ambient or elevated temperature.

Any excess cross-linking agent can be washed away. Water rinsing alone is typically insufficient to remove all excess cross-linking agent. Water rinsing can also be followed with or replaced by rinsing with a buffer and/or alcohol solvent, such as ethanol or isopropanol alcohol to remove the unreacted cross-linking agent (e.g. BDDE). It is contemplated that multiple washings may be necessary to remove all or substantially all of the excess cross-linking agent.

### III. Methods of Using the Biocompatible Mesh Compositions

The biocompatible mesh compositions of the present invention can be used for all surgical applications wehere synthetic (permanent and resorbable) mesh compositions are currently being used. For example, in addition to hernia repair, the biocompatible mesh compositions can also be used in breast reconstruction applications.

The biocompatible mesh compositions described herein can be used to treat any of a wide range of disorders in which amelioration or repair of tissue is needed. Tissue defects can arise from diverse medical conditions, including, for example, congenital malformations, traumatic injuries, infections, and oncologic resections. Thus, the biocompatible mesh compositions can be used to repair defects in any soft tissue, e.g., tissues that connect, support, or surround other structures and organs of the body. The biocompatible mesh compositions can also be used in support of bone repair, e.g., as a periosteal graft to support bone or an articular graft to drive cartilage repair. Soft tissue can be any non-osseous tissue. Soft tissue can also be epithelial tissue, which covers the outside of the body and lines the organs and cavities within the body. Examples of epithelial tissue include, but are not limited to, simple squamous epithelia, stratified squamous epithelia, cuboidal epithelia, or columnar epithelia.

Soft tissue can also be connective tissue, which functions to bind and support other tissues. One example of connective tissue is loose connective tissue (also known as areolar connective tissue). Loose connective tissue, which functions to bind epithelia to underlying tissues and to hold organs in place, is the most widely distributed connective tissue type in vertebrates. It can be found in the skin beneath the dermis layer; in places that connect epithelium to other tissues; underneath the epithelial tissue of all the body systems that have external openings; within the mucus membranes of the digestive, respiratory, reproductive, and urinary systems; and surrounding the blood vessels and nerves. Loose connective tissue is named for the loose "weave" of its constituent fibers which include collagenous fibers, elastic fibers (long, thread-like stretchable fibers composed of the protein clastin) and reticular fibers (branched fibers consisting of one or more types of very thin collagen fibers). Connective tissue can also be fibrous connective tissue, such as tendons, which attach muscles to bone, and ligaments, which joint bones together at the joints. Fibrous connective tissue is composed primarily of tightly packed collagenous fibers, an arrangement that maximizes tensile strength. Soft tissue can also be muscle tissue. Muscle tissue includes: skeletal muscle, which is responsible for voluntary movements; smooth muscle, which is found in the walls of the digestive tract, bladder arteries and other internal organs; and cardiac muscle, which forms the contractile wall of the heart.

The biocompatible mesh compositions can be used to repair soft tissues in many different organ systems that fulfill a range of physiological functions in the body. These organ systems can include, but are not limited to, the muscular system, the genitourinary system, the gastroenterological system, the integumentary system, the circulatory system and the respiratory system. The compositions are particularly useful for repairs to connective tissue, including the fascia, a specialized layer that surrounds muscles, bones and joints, of the chest and abdominal wall and for repair and reinforcement of tissue weaknesses in urological, gynecological and gastroenterological anatomy.

The biocompatible mesh compositions are highly suitable for hernia repair or any abdominal wall defect (e.g., defect in fascia due to trauma, disease, surgery, anatomic abnormalities, etc.). A hernia is the protrusion of the contents of a body cavity out of the body cavity in which the contents are normally found. These contents are often enclosed in the thin membrane that lines the inside of the body cavity; together the membrane and contents are referred to as a "hernial sac". Most commonly hernias develop in the abdomen, when a weakness in the abdominal wall expands into a localized hole or defect through which the intestinal protrusion occurs. These weaknesses in the abdominal wall typically occur in locations of natural thinning of the abdominal wall, that is, at sites where there are natural openings to allow the passage of canals for the blood vessels that extend from the abdomen to the extremities and other organs. Other areas of potential weakness are sites of any previous abdominal surgery. Fatty tissue usually enters a hernia first, but it can be followed by a segment of intestine or other intraabdominal organ. If a segment of internal organ becomes trapped within the hernia sac such that the blood supply to the organ is impaired, the patient is at risk for serious complications including intestinal blockage, gangrene, and death. Hernias do not heal spontaneously and often increase in size over time, so that surgical repair is necessary to correct the condition. In general, hernias are repaired by reinserting the hernia sac back into the body cavity followed by repair of the weakened muscle tissue.

There are many kinds of hernias. With the exception of inguinal and scrotal hernias, which are only present in males, hernias can be found in individuals of any age or gender. Examples of hernias include: direct inguinal hernias, in which the intestine can bulge into the inguinal canal via the back wall of the inguinal canal; indirect inguinal hernias, in which the intestine can bulge into the inguinal canal via a weakness at the apex of the inguinal canal; fermoral hernias, in which the abdominal contents pass into the weak area created by the passage of the femoral blood vessels into the lower extremities; scrotal hernias, in which the intestinal contents bulge into the scrotum; Spigelian hernia, in which the hernia occurs along the edge of the rectus abdominus muscle; obturator hernia, in which the abdominal contents (e.g., intestine or other abdominal organs) protrude into the obturator canal, lumbar hernias, e.g., Petit's hernia, in which the hernia is through Petit's triangle, the inferior lumbar triangle, and Grynfeltt's hernia, in which the hernia is through Grynfeltt-Lesshaft triangle, the superior lumbar triangle; Richter's hernia, in which only one sidewall of the bowel becomes strangulated; Hesselbach's hernia, in which the hernia is through Hesselbach's triangle; pantaloon hernia, in which the hernia sac protrudes on either side of the inferior epigastric vessels to give a combined direct and indirect inguinal hernia; Cooper's hernia; epigastric hernia (in which the hernia occurs between the navel and the lower part of the rib cage in the midline of the abdomen); diaphragmatic or hiatal hernias, e.g., Bochdalek's hernia and Morgagni's hernia, in which a portion of the stomach protrudes through the diaphragmatic esophageal hiatus; and umbilical hernia, in which the protrusion is through the navel.

In contrast to hernias of congenital origin, incisional hernias, also known as ventral or recurrent hernias, occur in the abdomen in the area of an old surgical scar. Incisional hernias have a higher risk of returning after surgical repair than do congenital hernias. Moreover, in the case of multiple recurrent hernias, i.e., hernias that recur after two or more repairs have been carried out, the likelihood of successful repair decreases with each subsequent procedure.

The biocompatible mesh compositions can be used to treat other medical conditions that result from tissue weakness. One condition for which the biocompatible mesh compositions are useful is in the repair of organ prolapse. Prolapse is a condition in which an organ, or part of an organ, falls or slips out of place. Prolapse typically results from tissue weakness that can stem from either congenital factors, trauma or disease. Pelvic organ prolapse can include prolapse of one or more organs within the pelvic girdle; tissue weakening due to pregnancy, labor and childbirth is a common cause of the condition in women. Examples of organs involved in pelvic organ prolapse include the bladder (cyctocele), which can prolapse into the vagina; the urethra, which can prolapse into the vagina; the uterus, which can prolapse into the vagina; the small intestine (enterocele), which can prolapse against the wall of the vagina; the rectum (rectocele), which can prolapse against the wall of the vagina; and vaginal prolapse, in which a portion of the vaginal canal can protrude from the opening of the vagina. Depending upon the organ involved and the severity of the prolapse, patients with pelvic organ prolapse may experience pain upon sexual intercourse, urinary frequency, urinary incontinence, urinary tract infection, renal damage, and constipation. Remedies include both non-surgical and surgical options; in severe cases, reconstruction of the tissues of the pelvic floor, i.e., the muscle fibers and connective tissue that span the area underneath the pelvis and provides support for the pelvic organs, e.g., the bladder, lower intestines, and the uterus (in women) may be required.

The biocompatible mesh compositions are also useful in repairs of the gastrointestinal system. Esophageal conditions in need of repair include, but are not limited to, traumatic rupture of the esophagus, e.g., Boerhaave syndrome, Mallory-Weiss syndrome, trauma associated with iatrogenic esophageal perforation that may occur as a complication of an endoscopic procedure or insertion of a feeding tube or unrelated surgery; repair of congenital esophageal defects, e.g., esophageal atresia; and oncologic esophageal resection.

The biocompatible mesh compositions can be used to repair tissues that have never been repaired before or they can be used to repair tissues that have been treated one or more times with biocompatible mesh compositions or with other methods known in the art or they can be used along with other methods of tissue repair including suturing, tissue grafting, or synthetic tissue repair materials.

The biocompatible mesh compositions can be applied to an individual in need of treatment using techniques known to those of skill in the art. The biocompatible mesh compositions can be: (a) wrapped around a tissue that is damaged or that contains a defect; (b) placed on the surface of a tissue that is damaged or has a defect; (c) rolled up and inserted into a cavity, gap, or space in the tissue. One or more (e.g., one, two, three, four, five, six, seven, eight. nine, ten, 12, 14, 16, 18, 20, 25, 30, or more) such biocompatible mesh compositions , stacked or adjacent to each other, can be used at any particular site. The biocompatible mesh compositions can be held in place by, for example, sutures, staples, tacks, or tissue glues or sealants known in the art. Alternatively, if, for example, packed sufficiently tightly into a defect or cavity, they may need no securing device.

The following examples are provided to better explain the various embodiments and should not be interpreted in any way to limit the scope of the present disclosure

### EXEMPLIFICATION

### Example 1 Preparation of plasma treated mesh with primary hydroxyl (-OH) groups

Polypropylene (PP) mesh was cleaned by immersion in ethanol overnight. The cleaned mesh was then placed in a commercial plasma treatment chamber. After vacuum evacuating, the chamber was filled with argon gas mixed with allyl alcohol. Plasma was generated by a radio frequency generator (13.5 MHz, input power at 100 watts). The polymer mesh was treated with energized plasma gas for 120 seconds, resulting in surface modification with primary -OH(-CH₂CH₂OH) groups.

### Example 2 Preparation of HA covalent coating on a mesh

Hyaluronic acid sodium salt from Streptococcus equi (MW ∼ 1.6 MD, sigma) was dissolved in 0.25 M NaOH at a concentration of 50 mg/ml. After the HA solution became homogenous, a hydroxyl containing mesh (cellulose, silk, or a plasma treated polymer mesh with -OH functional groups), was added and completely immersed into the solution. 2 µL 1,4-butanediol diglycidyl ether (BDDE) per 1 ml HA solution (HA:BDDE molar ratio is about 12:1) was added into the mixture and vortexed briefly. Next, the mixture was incubated at 37 °C for 1 hr. The mixture was then dried under vacuum at 37 °C for 30 mins or longer. The completely dried hydrogel mesh composite was then washed in 3:2 mix ratio of IPA/H₂O solution with three solution changes, including one overnight wash. The mesh is further washed with H₂O extensively.

### CLAUSES

Representative features are set out in the following clauses, which stand alone or may be combined, in any combination, with one or more features disclosed in the text and/or drawings of the specification.
1. A biocompatible mesh composition comprising:
   a mesh having a multi-layered molecular coating of hyaluronic acids, wherein the primary hydroxyl (-OH) groups of hyaluronic acids are cross-linked with the -OH containing groups on the mesh via a homobifunctional cross-linking agent, and the primary hydroxyl (-OH) groups of hyaluronic acids are also cross-linked to each other via the homobifunctional cross-linking agent.
2. The biocompatible mesh composition of clause 1, wherein the homobifunctional cross-linking agent is butanediol diglycidyl ether (BDDE), 1, 2, 7, 8-diepoxyoctane (DEO), glycerol diglycidyl ether, or divinyl sulfone (DVS).
3. The biocompatible mesh composition of clause 1 or 2, wherein the mesh is polystyrene, polyethylene, polypropylene, polyethylene terephthalate, polytefrafluoroethylene, polylactide, cellulose or silk.
4. The biocompatible mesh composition of any one of clauses 1-3, wherein the -OH containing groups on the mesh are represented by -RCH₂OH, wherein R is C₁-C₆ alkylene or R is absent.
5. The biocompatible mesh composition of clause 4, wherein the -OH containing groups are -CH₂OH or -CH₂CH₂CH₂OH.
6. The biocompatible mesh composition of any one of clauses 1-5, wherein the hyaluronic acid has a molecular weight in a range of from about 350,000 daltons to about 2,000,000 daltons.
7. The biocompatible mesh composition of any one of clauses 1-6, wherein the mesh is silk and the homobifunctional cross-linking agent is butanediol diglycidyl ether (BDDE).
8. The biocompatible mesh composition of any one of clauses 1-6, wherein the mesh is cellulose and the homobifunctional cross-linking agent is butanediol diglycidyl ether (BDDE).
9. The biocompatible mesh composition of any one of clauses 1-6, wherein the mesh is polypropylene and the homobifunctional cross-linking agent is butanediol diglycidyl ether (BDDE).
10. The biocompatible mesh composition of any one of clauses 1-9, wherein the molar ratio between the hyaluronic acid and the homobifunctional cross-linking agent is 20:1 to 1:1.
11. The biocompatible mesh composition of any one of clauses 1-9, wherein the molar ratio between the hyaluronic acid and the homobifunctional cross-linking agent is 15:1 to 1:1.
12. The biocompatible mesh composition of any one of clauses 1-11, wherein the mesh is in the form of a flexible sheet.
13. A process of making a biocompatible mesh composition, the method comprising:
   i) treating a mesh with plasma to form a mesh with -OH containing groups on its surface;
   ii) contacting the mesh with -OH containing groups with a solution containing hyaluronic acids and a homobifunctional cross-linking agent to form a biocompatible mesh composition in which the mesh has a multi-layered molecular coating of hyaluronic acids such that the primary hydroxyl (-OH) groups of hyaluronic acids are cross-linked with the - OH containing groups on the mesh via the homobifunctional cross-linking agent, and the primary hydroxyl (-OH) groups of hyaluronic acids are also cross-linked to each other via the homobifunctional cross-linking agent.
14. A process of making a biocompatible mesh composition, the method comprising:
   contacting a mesh with -OH containing groups on its surface with a solution containing hyaluronic acid and a homobifunctional cross-linking agent, to form a biocompatible mesh composition in which the mesh has a multi-layered molecular coating of hyaluronic acids such that the primary hydroxyl (-OH) groups of hyaluronic acids are cross-linked with the -OH containing groups on the mesh via the homobifunctional cross-linking agent, and the primary hydroxyl (-OH) groups of hyaluronic acids are also cross-linked to each other via the homobifunctional cross-linking agent.
15. The process of clause 13 or 14, wherein the mesh is polystyrene, polyethylene, polypropylene, polyethylene terephthalate, polytefrafluoroethylene, polylactide, cellulose or silk.
16. The process of any one of clauses 13-15, wherein the process comprises a further step of allowing the biocompatible mesh composition to dry.
17. The process of any one of clauses 13-16, wherein the homobifunctional cross-linking agent is butanediol diglycidyl ether (BDDE), 1, 2, 7, 8-diepoxyoctane (DEO), glycerol diglycidyl ether, or divinyl sulfone (DVS).
18. The process of any one of clauses 13-17, wherein the concentration of the hyaluronic acid is between 5 mg/mL and 50 mg/mL.
19. The process of any one of clauses 13-17, wherein the concentration of the hyaluronic acid is between 25 mg/mL and 50 mg/mL.
20. The process of any one of clauses 13-19, wherein the hyaluronic acid has a molecular weight in a range of from about 350,000 daltons to about 2,000,000 daltons.
21. The process of any one of clauses 13-20, wherein the molar ratio between the hyaluronic acid and the homobifunctional cross-linking agent is 20:1 to 1:1.
22. The process of any one of clauses 13-20, wherein the molar ratio between the hyaluronic acid and the homobifunctional cross-linking agent is 15:1 to 1:1.
23. The process of any one of clauses 13-22, wherein the mesh is silk and the homobifunctional cross-linking agent is butanediol diglycidyl ether (BDDE).
24. The process of any one of clauses 13-22, wherein the mesh is cellulose and the homobifunctional cross-linking agent is butanediol diglycidyl ether (BDDE).
25. The process of any one of clauses 13-22, wherein the mesh is polypropylene and the homobifunctional cross-linking agent is butanediol diglycidyl ether (BDDE).
26. The process of any one of clauses 13 and 15-25, wherein the plasma treatment is in the presence of allyl alcohol.
27. The process of any one of clauses 13-26, wherein the mesh in the form of a flexible sheet.
28. A biocompatible mesh composition formed by the process of any one of clauses 13-27.

## Claims

1. A biocompatible mesh composition comprising:
a mesh having a multi-layered molecular coating of hyaluronic acids, wherein the primary hydroxyl (-OH) groups of hyaluronic acids are cross-linked with the -OH containing groups on the mesh via a homobifunctional cross-linking agent, and the primary hydroxyl (-OH) groups of hyaluronic acids are also cross-linked to each other via the homobifunctional cross-linking agent,
wherein the mesh is polypropylene, silk, or cellulose, and the homobifunctional cross-linking agent is butanediol diglycidyl ether (BDDE).

2. The biocompatible mesh composition of claim 1, wherein the -OH containing groups on the mesh are represented by -RCH₂OH, wherein R is C₁-C₆ alkylene or R is absent, preferably, the -OH containing groups are -CH₂OH or -CH₂CH₂CH₂OH.

3. The biocompatible mesh composition of claim 1 or 2, wherein the hyaluronic acid has a molecular weight in a range of from about 350,000 daltons to about 2,000,000 daltons.

4. The biocompatible mesh composition of any one of claims 1-3, wherein the molar ratio between the hyaluronic acid and the homobifunctional cross-linking agent is 20:1 to 1:1, preferably, 15:1 to 1:1.

5. The biocompatible mesh composition of any one of claims 1-4, wherein the mesh is in the form of a flexible sheet.

6. A process of making a biocompatible mesh composition, the method comprising:
contacting a mesh with -OH containing groups on its surface with a solution containing hyaluronic acid and a homobifunctional cross-linking agent, to form a biocompatible mesh composition in which the mesh has a multi-layered molecular coating of hyaluronic acids such that the primary hydroxyl (-OH) groups of hyaluronic acids are cross-linked with the -OH containing groups on the mesh via the homobifunctional cross-linking agent, and the primary hydroxyl (-OH) groups of hyaluronic acids are also cross-linked to each other via the homobifunctional cross-linking agent,
wherein the mesh is polypropylene, silk, or cellulose, and the homobifunctional cross-linking agent is butanediol diglycidyl ether (BDDE).

7. The process of claim 6, further comprising:
treating a mesh with plasma to form the mesh with -OH containing groups on its surface.

8. The process of claim 6 or 7, wherein the process comprises a further step of allowing the biocompatible mesh composition to dry.

9. The process of any one of claims 6-8, wherein the concentration of the hyaluronic acid is between 5 mg/mL and 50 mg/mL, preferably, between 25 mg/mL and 50 mg/mL.

10. The process of any one of claims 6-9, wherein the hyaluronic acid has a molecular weight in a range of from about 350,000 daltons to about 2,000,000 daltons.

11. The process of any one of claims 6-10, wherein the molar ratio between the hyaluronic acid and the homobifunctional cross-linking agent is 20:1 to 1:1, preferably, 15:1 to 1:1.

12. The process of any one of claims 6-11, wherein the plasma treatment is in the presence of allyl alcohol.

13. The process of any one of claims 6-12, wherein the mesh in the form of a flexible sheet.

14. A biocompatible mesh composition formed by the process of any one of claims 6-13.
